# EUROPÄISCHE PATENTSCHRIFT

(11) **EP 3 837 542 B1**
(45) Veröffentlichungstag und Bekanntmachung des Hinweises auf die Patenterteilung: **06.03.2024**
(21) Anmeldenummer: 19753301.1
(22) Anmeldetag: 06.08.2019
(51) Int. Cl.: G01N 27/87, G01N 27/85, A61M 5/32, G01B 7/312

(54) **VERFAHREN UND VORRICHTUNG ZUR INSPEKTION EINES ZUSTANDS EINER AUF EINER SPRITZE AUFGESETZTEN KANÜLE**
METHOD AND DEVICE FOR THE INSPECTION OF A CONDITION OF A CANNULA PLACED ON A SYRINGE
PROCÉDÉ ET DISPOSITIF D'INSPECTION D'UN ÉTAT D'UNE CANULE MISE EN PLACE SUR UNE SERINGUE

(30) Priorität: 16.08.2018 CH 9952018
(43) Veröffentlichungstag der Anmeldung: 23.06.2021
(73) Patentinhaber: Wilco AG, 5610 Wohlen (CH)
(72) Erfinder: KAHL, Matthias, 79541 Lörrach (DE); STIRNIMANN, Christian, 8157 Dielsdorf (CH)
(74) Vertreter: Troesch Scheidegger Werner AG
(86) Internationale Anmeldenummer: PCT/EP2019/071144
(87) Internationale Veröffentlichungsnummer: WO 2020/035355

(56) Entgegenhaltungen:
- EP-A2- 2 763 722
- DE-A1-102007 040 488
- DE-C1- 19 806 971
- US-A1- 2012 307 972
- US-A1- 2013 242 082

## Beschreibung

### TECHNISCHES GEBIET DER ERFINDUNG

Die vorliegende Erfindung betrifft Verfahren zur Inspektion eines Zustands, insbesondere zur Erkennung von Defekten, einer auf einer Spritze aufgesetzten Kanüle (bzw. Spritzennadel) und Vorrichtungen zur Ausführung solcher Verfahren.

### HINTERGRUND DER ERFINDUNG

Bei der Herstellung von medizinischen Spritzen kann es beim Aufsetzten der nicht durchsichtigen Schutzkappe (engl. Needle Shield) dazu kommen, dass sich die darunterliegende Kanüle bzw. Nadel verbiegt oder gestaucht wird, diese durch die Schutzkappe sticht oder sogar bricht. So wird die Kanüle nicht nur eine Gefahr für den Benutzer, sondern kann auch ihre Sterilität verlieren. Heute werden unterschiedliche Methoden zum Erkennen solcher Schäden eingesetzt. So wird etwa die klassische Bildverarbeitung verwendet, um die Stellung der Schutzkappe zu erkennen. Dieses Verfahren sortiert jedoch einen relativ hohen Anteil an guten Spritzen aus (hohe "False Rejection Rate"). Die derzeit wohl zuverlässigste Methode beruht auf dem Röntgen jeder Spritze und auf der automatischen Analyse der aufgenommenen Röntgenbilder. Diese Technologie ist aber ziemlich aufwändig, so muss z.B. der Röntgenbereich gegen aussen abgeschirmt sein, was sehr kostenintensiv ist. Zudem kann das Produkt durch die Röntgenstrahlung belastet werden. Alternativ gibt es die Hochspannungsprüfung. Dabei wird eine unter Hochspannung stehende Elektrode an das Ende mit der Schutzkappe gehalten. Wenn die Kanüle durch die Schutzkappe sticht, ist dies mit der Hochspannungsprüfung erkennbar. Verbogene, gestauchte oder gebrochenen Kanülen, die sich unter einer intakten Schutzkappe befinden, sind jedoch nicht zuverlässig zu erkennen.

Es besteht daher der Bedarf nach Mitteln, welche eine einfache (und folglich kostengünstige) sowie zuverlässige Erkennung von Defekten an einer auf einer Spritze aufgesetzten Kanüle ermöglichen. Um in Inspektionsmaschinen mit einem hohen Durchsatz von Prüflingen, wie z.B. um 600 Spritzen pro Minute, eingesetzt werden zu können, müssen die Mittel zudem in der Lage sein, die einzelnen Prüflinge sehr rasch mit hoher Zuverlässigkeit prüfen zu können.

EP 2 763 722 A2 betrifft ein System zur Bestimmung der Position eines Elements, wobei das System eine Sensoranordnung und ein bewegliches Element mit einem eingebauten Magnet, das relativ zur Sensoranordnung bewegbar ist, um ein räumliches Magnetfeld zu erzeugen, umfasst. Die Sensoranordnung weist einen oder mehrere Sensoren auf, von denen jeder ein Magnetfeld entlang von drei Achsen messen kann. Ein Prozessor bestimmt anhand des von der Sensoranordnung gemessenen Magnetfelds eine Position des beweglichen Elements relativ zu einer gegebenen Position.

US 2012/0307972 A1 beschreibt eine Inspektionsvorrichtung sowie ein Verfahren zur elektromagnetischen Überprüfung der Kanülenausrichtung in einem an einer Spritze befestigten Kanülenschutzkappe. Die Inspektionsvorrichtung umfasst einen elektromagnetischen Bildgeber mit einem Emitter und einem Detektor sowie einen Bildanalysator.

US 2013/0242082 A1 offenbart ein System zum Anbringen eines Nadelschutzes an einer Spritze, wobei das System eine Inspektionskamera umfasst, die in der Nähe der Spritzenachse positioniert und dazu konfiguriert ist, visuell Informationen in Bezug auf die Positionierung des Nadelschutzes relativ zu der Spritze zu erfassen.

DE 198 06 971 C1 beschreibt ein Verfahren zur berührungslosen Überwachung auf vorgeschriebenen Sitz einer auf die Kanüle einer medizinischen Spritze aufgesetzten Nadelschutzkappe sowie eine Vorrichtung zur Durchführung des Verfahrens. Dazu wird an die Kanüle einerseits und an eine die Nadelschutzkappe umgebende Gegenelektrode andererseits eine Hochspannung angelegt und der in einer der elektrischen Zuleitungen zur Kanüle bzw. zur Gegenelektrode fliessende Strom überwacht, wobei bei Überschreitung eines vorgegebenen Stromwertes die der Prüfung unterzogene Spritze als fehlerhaft gekennzeichnet wird.

DE 10 2007 040 488 A1 betrifft eine Röntgeneinrichtung zur Untersuchung von eine Kanüle aufweisenden Spritzenkappen, mit einer Röntgenstrahlungsquelle, einem Röntgendetektor und einer die Spritzenkappe im Strahlengang an einem Untersuchungsort haltenden Halterung.

### ZUSAMMENFASSUNG DER ERFINDUNG

Es ist eine Aufgabe der vorliegenden Erfindung ein Verfahren zur raschen, zuverlässigen und kostengünstigen Inspektion eines Zustands, insbesondere zur Erkennung von Defekten, einer auf einer Spritze aufgesetzten Kanüle bereitzustellen. Diese Aufgabe wird erfindungsgemäss durch das in Anspruch 1 festgelegte Prüfverfahren erfüllt.

Es ist zudem eine Aufgabe der vorliegenden Erfindung eine entsprechende Vorrichtung zur raschen, zuverlässigen und kostengünstigen Inspektion eines Zustands, insbesondere zur Erkennung von Defekten, einer auf einer Spritze aufgesetzten Kanüle vorzuschlagen. Diese Aufgabe wird erfindungsgemäss durch die in Anspruch 9 festgelegte Prüfvorrichtung gelöst.

Spezifische erfindungsgemässe Ausführungsvarianten werden in den abhängigen Ansprüchen angegeben.

Ein erfindungsgemässes Verfahren zur Inspektion eines Zustands, insbesondere zum Erkennen von bestimmten Defekten, einer auf einer Spritze aufgesetzten Kanüle, welche sich in einer Kanülenschutzkappe befindet, umfasst ein Messen eines magnetischen Feldes, insbesondere einer magnetischen Feldverteilung, in einer Umgebung der Kanüle, und ein Bestimmen einer Verformung des magnetischen Feldes, insbesondere eines Feldlinienverlaufs, im Vergleich zum Fall einer geraden Kanüle.

In einer Ausführungsvariante des Verfahrens werden zum Messen des magnetischen Feldes, insbesondere der magnetischen Feldverteilung, ein oder mehrere Magnetfeldsensoren, insbesondere ein Induktionssensor, ein Fluxgate-Magnetometer, ein Hall-Sensor, ein magnetoresistiver Sensor, wie z.B. ein AMR- (engl. "anisotrope magnetoresistive"), CMR- (engl. "colossal magnetoresistance "), GMR- (engl. "giant magnetoresistance") oder TMR- (engl. "tunnel magnetoresistance") Sensor, eingesetzt.

In einer weiteren Ausführungsvariante des Verfahrens sind der eine oder die mehreren Magnetfeldsensoren als ein-, zwei- oder drei-achsige Magnetfeldsensoren ausgeführt, um das magnetische Feld, insbesondere die magnetische Feldverteilung, ein-, zwei- oder drei-dimensional auszumessen.

In einer weiteren Ausführungsvariante des Verfahrens wird zum Messen des magnetischen Feldes, insbesondere der magnetischen Feldverteilung, die Kanüle relativ zu dem einen oder den mehreren Magnetfeldsensoren bewegt, wobei die Spritze insbesondere um ihre Längsachse rotiert und/oder entlang ihrer Längsachse bewegt wird, oder der eine oder die mehreren Magnetfeldsensoren parallel zur Längsachse der Spritze bewegt werden.

In einer weiteren Ausführungsvariante des Verfahrens werden ein oder mehrere Motoren, wie z.B. Servo- oder Schrittmotoren, zum Rotieren und/oder zum Längsbewegen der Spritze eingesetzt, wobei ein Getriebe eingesetzt wird, sodass die Drehfrequenz des einen oder der mehreren Motoren ein Vielfaches oder ein Bruchteil der Rotationsfrequenz der Spritze ist. Dadurch wird verhindert, dass Magnetfelder, welche von dem einen oder den mehreren Motoren erzeugt werden, das in der Umgebung der Kanüle zu messende magnetische Feld, bzw. die zu messende magnetische Feldverteilung, stören.

In einer weiteren Ausführungsvariante des Verfahrens wird das magnetische Feld, mindestens zum Teil durch das Erdmagnetfeld, durch eine Anordnung mit einem oder mehreren Permanentmagneten gebildetes Magnetfeld, oder durch eine Anordnung mit einem oder mehreren Elektromagneten, wie z.B. einer Helmholtz-Spule, gebildetes Magnetfeld, erzeugt, wobei die Anordnung insbesondere auch einen oder mehrere Eisenkerne aufweist.

In einer weiteren Ausführungsvariante des Verfahrens wird eine magnetische Abschirmung zur Unterdrückung von magnetischen Störfeldern eingesetzt.

In einer weiteren Ausführungsvariante umfasst das Verfahren ein Vergleichen des gemessenen magnetischen Feldes, insbesondere der gemessenen magnetischen Feldverteilung, für die Kanüle mit dem gemessenen magnetischen Feld, insbesondere der gemessenen magnetischen Feldverteilung, für eine Referenzspritze mit einer aufgesetzten Referenzkanüle, welche sich in einer Kanülenschutzkappe befindet, welche einen Sollzustand aufweist, und ein Bestimmen des Zustands der auf der Spritze aufgesetzten Kanüle basierend auf dem Vergleichen.

In einer weiteren Ausführungsvariante des Verfahrens basiert das Bestimmen des Zustands der auf der Spritze aufgesetzten Kanüle auf einem Vergleichen einer Amplitude und/oder Phasenlage des gemessenen magnetischen Feldes für die Referenzkanüle mit einer Amplitude und/oder Phasenlage des gemessenen magnetischen Feldes für die Kanüle.

In einer weiteren Ausführungsvariante des Verfahrens basiert das Bestimmen des Zustands der auf der Spritze aufgesetzten Kanüle auf einem Vergleichen einer Amplitude und/oder Phasenlage des gemessenen magnetischen Feldes an zwei unterschiedlichen Orten, welches z.B. mit zwei zueinander versetzt angeordneten Magnetfeldsensoren gemessen wird.

In einer weiteren Ausführungsvariante umfasst das Verfahren ein Transformieren eines gemessenen zeitlichen Verlaufs des magnetischen Feldes, insbesondere der magnetischen Feldverteilung, vom Zeitbereich in den Frequenzbereich.

In einer weiteren Ausführungsvariante umfasst das Verfahren ein Bestimmen einer Frequenzkomponente des magnetischen Feldes, insbesondere der magnetischen Feldverteilung, bei einer Rotationsfrequenz der Spritze, insbesondere auch bei der doppelten Rotationsfrequenz der Spritze, z.B. mittels einer diskreten Fouriertransformation (DFT).

In einer weiteren Ausführungsvariante des Verfahrens ist der Zustand der Kanüle mindestens eines von gerade, verbogen, geknickt, gestaucht, ab-/gebrochen, koaxial zur Spritzenlängsachse, schräg zur Spritzenlängsachse, exzentrisch zur Spritzenlängsachse.

Gemäss einem weiteren Aspekt der vorliegenden Erfindung umfasst eine Vorrichtung zur Inspektion eines Zustands einer auf einer Spritze aufgesetzten Kanüle, welche sich in einer Kanülenschutzkappe befindet, ein oder mehrere Magnetfeldsensoren, insbesondere ein Induktionssensor, ein Fluxgate-Magnetometer, ein Hall-Sensor, ein magnetoresistiver Sensor, wie z.B. ein AMR- (engl. "anisotrope magnetoresistive"), CMR- (engl. "colossal magnetoresistance "), GMR- (engl. "giant magnetoresistance") oder TMR- (engl. "tunnel magnetoresistance") Sensor, zum Messen eines magnetischen Feldes, insbesondere einer magnetischen Feldverteilung, in einer Umgebung der Kanüle. Die Vorrichtung umfasst weiter eine Vergleichereinheit, wobei die Vergleichereinheit zum Vergleichen des gemessenen magnetischen Feldes, insbesondere der gemessenen magnetischen Feldverteilung, für die Kanüle mit dem gemessenen magnetischen Feld, insbesondere der gemessenen magnetischen Feldverteilung, für eine Referenzspritze mit einer aufgesetzten Referenzkanüle, welche sich in einer Kanülenschutzkappe befindet, welche einen Sollzustand aufweist, ausgebildet ist, und wobei die Vergleichereinheit ausgebildet ist, den Zustand der auf der Spritze aufgesetzten Kanüle basierend auf dem Vergleich zu bestimmen.

In einer weiteren Ausführungsvariante der Vorrichtung sind der eine oder die mehreren Magnetfeldsensoren als ein-, zwei- oder drei-achsige Magnetfeldsensoren ausgeführt, um das magnetische Feld, insbesondere die magnetische Feldverteilung, ein-, zwei- oder drei-dimensional auszumessen.

In einer weiteren Ausführungsvariante umfasst die Vorrichtung weiter eine Einrichtung zum Bewegen der Kanüle relativ zu dem einen oder den mehreren Magnetfeldsensoren, insbesondere zum Rotieren die Spritze um ihre Längsachse und/oder zum Fahren der Spritze entlang ihrer Längsachse, oder zum Fahren des einen oder der mehreren Magnetfeldsensoren parallel zur Längsachse der Spritze.

In einer weiteren Ausführungsvariante umfasst die Vorrichtung weiter ein oder mehrere Motoren, wie z.B. Servo- oder Schrittmotoren, zum Rotieren und/oder zum Längsbewegen der Spritze, wobei ein Getriebe eingesetzt wird, sodass die Drehfrequenz des einen oder der mehreren Motoren ein Vielfaches oder ein Bruchteil der Rotationsfrequenz der Spritze ist.

In einer weiteren Ausführungsvariante umfasst die Vorrichtung weiter eine Anordnung mit einem oder mehreren Permanentmagneten und/oder eine Anordnung mit einem oder mehreren Elektromagneten, wie z.B. einer Helmholtz-Spule, zum Erzeugen eines Magnetfeldes, wobei die Anordnung insbesondere auch einen oder mehrere Eisenkerne aufweist.

In einer weiteren Ausführungsvariante umfasst die Vorrichtung weiter eine magnetische Abschirmung zur Unterdrückung von magnetischen Störfeldern.

In einer weiteren Ausführungsvariante der Vorrichtung ist die Vergleichereinheit ausgebildet, um einen Vergleich einer Amplitude und/oder Phasenlage des gemessenen magnetischen Feldes für die Referenzkanüle mit einer Amplitude und/oder Phasenlage des gemessenen magnetischen Feldes für die Kanüle auszuführen, und daraus den Zustand der auf der Spritze aufgesetzten Kanüle zu bestimmen.

In einer weiteren Ausführungsvariante umfasst die Vorrichtung weiter eine Einheit zur Ausführung einer Transformation eines gemessenen zeitlichen Verlaufs des magnetischen Feldes, insbesondere der magnetischen Feldverteilung, vom Zeitbereich in den Frequenzbereich.

In einer weiteren Ausführungsvariante umfasst die Vorrichtung weiter einen Ausgang, insbesondere zum Bereitstellen eines Ausgangssignals, welcher dazu ausgebildet ist, um den Zustand der Kanüle als mindestens eines von gerade, verbogen, geknickt, gestaucht, ab-/ gebrochen, koaxial zur Spritzenlängsachse, schräg zur Spritzenlängsachse, exzentrisch zur Spritzenlängsachse anzugeben.

In einer weiteren Ausführungsvariante der Vorrichtung sind mehrere Magnetfeldsensoren vertikal übereinander entlang einer Achse, insbesondere äquidistant beabstandet, angeordnet, wobei die Achse seitlich parallel zur Längsachse der zu inspizierenden Spritze angeordnet ist.

In einer weiteren Ausführungsvariante umfasst die Vorrichtung eine Helmholtz-Spule bestehend aus zwei koaxial angeordneten Spulen, deren Spulenachse horizontal angeordnet ist, um das magnetische Feld in horizontaler Richtung zu erzeugen, wobei die Spritze für die Inspektion zwischen die beiden Spulen angeordnet wird.

Es sei angemerkt, dass Kombinationen der oben genannten Ausführungsvarianten möglich sind, die wiederum zu spezifischeren Ausführungsvarianten der vorliegenden Erfindung führen.

### KURZE BESCHREIBUNG DER ZEICHNUNGEN

Nichtlimitierende Ausführungsbeispiele der vorliegenden Erfindung werden nachstehend anhand von Figuren noch näher erläutert. Es zeigen:
- Fig. 1: ein Röntgenbild (gemäss Stand der Technik) einer Spritze mit aufgesetzter Schutzkappe, unter welcher sich eine verbogene Kanüle befindet;
- Fig. 2: ein Beispiel des Feldlinienverlaufs des Erdmagnetfeldes (mit einer Inklination von 64°) in der Umgebung einer gebogenen Spritzennadel aus Stahl;
- Fig. 3: schematisch eine seitliche Ansicht einer Ausführungsvariante einer erfindungsgemässen Vorrichtung; und
- Fig. 4: schematisch eine seitliche Ansicht einer weiteren Ausführungsvariante einer erfindungsgemässen Vorrichtung mit einer Helmholtz-Spule.

In den Figuren stehen gleiche Bezugszeichen für gleiche Elemente.

### DETAILLIERTE BESCHREIBUNG DER ERFINDUNG

Beim Montageprozess von medizinischen Spritzen, wie z.B. von Insulinspritzen für den einmaligen Gebrauch, wird die Kanüle bzw. Spritzennadel auf die Spritze aufgesetzt bzw. auf dieser befestigt, z.B. eingeklebt, und danach wird auf die Kanüle eine Schutzkappe angebracht. Dabei muss die Kanüle in das Material der Schutzkappe eindringen. Entsprechend läuft dieser Vorgang mit einer gewissen Kraft ab, sodass die Kanüle dabei z.B. verbogen, geknickt, gestaucht, (ab-)brechen kann. Diese Defekte stellen ein mehrfaches Risiko dar, so besteht Verletzungsgefahr bei der Benutzung und/oder die Sterilität ist nicht mehr gegeben.

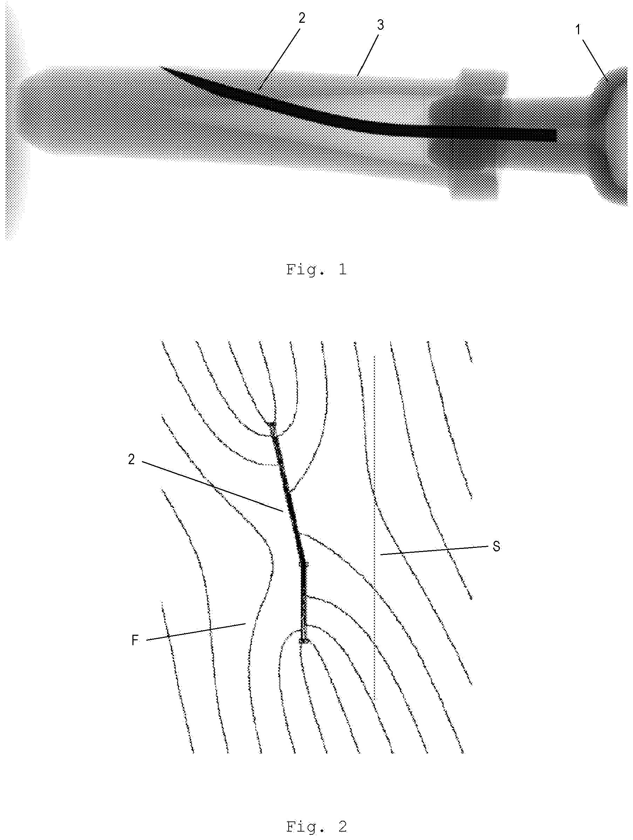

Fig. 1 zeigt ein Röntgenbild einer Spritze 1 mit aufgesetzter optisch intransparenten Schutzkappe 3, unter welcher sich eine verbogene Kanüle 2 befindet, wie es bei einem Verfahren gemäss dem Stand der Technik aufgenommen wird.

Bei der vorliegenden Erfindung wird der Effekt genutzt, dass ferromagnetische Materialen ein externes magnetisches Feld in ihrer Umgebung beeinflussen. So neigen ferromagnetische Materialien dazu magnetische Felder in sich hinein zu ziehen. Die Feldlinien eines externen magnetischen Feldes enden auf der Oberfläche des ferromagnetischen Körpers und verlaufen in dessen Innerem. Damit verursacht das Vorhandensein einer Spritzennadel aus Stahl eine lokale Veränderung des Verlaufs der magnetischen Feldlinien. Eine intakte, d.h. gerade Nadel wird eine andere Veränderung der magnetischen Feldlinien hervorrufen als eine Nadel, die z.B. einen Knick aufweist.

Fig. 2 illustriert schematisch ein Beispiel des Feldlinienverlaufs F des Erdmagnetfeldes in der Umgebung einer geknickten Spritzennadel bzw. Kanüle 2. Je nach Breitengrad neigt sich das Erdmagnetfeld unterschiedlich stark in Richtung der Erde. So weist das in Fig. 2 dargestellte Erdmagnetfeld entsprechend dem 47. Breitengrad eine Inklination von 64° nach unten auf. Die magnetischen Feldlinien F werden durch die Spritzennadel 2 aus Stahl gebündelt und die weiter aussenliegenden Feldlinien werden zur Spritzennadel 2 hin abgelenkt. Diese Verformung des Feldlinienverlaufs im Vergleich zum Fall, wo die Spritzennadel 2 gerade ist, kann mittels Messung des magnetischen Feldes, insbesondere der magnetischen Feldverteilung, in einer Umgebung der Kanüle 2 festgestellt werden. Basierend auf der Messung des magnetischen Feldes an einem oder mehreren Punkten, z.B. auf oder entlang der Messstrecke S, kann dann auf den Zustand der Kanüle 2 zurückgeschlossen werden, d.h. ob sie einen bestimmten Defekt wie z.B. einen Knick aufweist.

Zum Messen des magnetischen Feldes bzw. der magnetischen Feldverteilung können ein oder mehrere Magnetfeldsensoren, wie z.B. Induktionssensor, ein Fluxgate-Magnetometer, ein Hall-Sensor, ein magnetoresistiver Sensor, wie z.B. ein AMR- (engl. "anisotrope magnetoresistive"), CMR- (engl. "colossal magnetoresistance "), GMR- (engl. "giant magnetoresistance") oder TMR- (engl. "tunnel magnetoresistance") Sensor, eingesetzt werden. Diese Magnetfeldsensoren können als ein-, zwei- oder drei-achsige Magnetfeldsensoren ausgeführt sein, um das magnetische Feld bzw. die magnetische Feldverteilung ein-, zwei- oder drei-dimensional auszumessen. Dabei kann die Kanüle 2 relativ zu einem oder mehreren Magnetfeldsensoren bewegt werden, oder umgekehrt ein oder mehrere Magnetfeldsensoren können relativ zur Kanüle 2 bewegt werden, z.B. vertikal entlang der Messstrecke S, sodass das magnetische Feld bzw. die magnetische Feldverteilung über die ganze Länge der Kanüle 2 bestimmt werden kann.

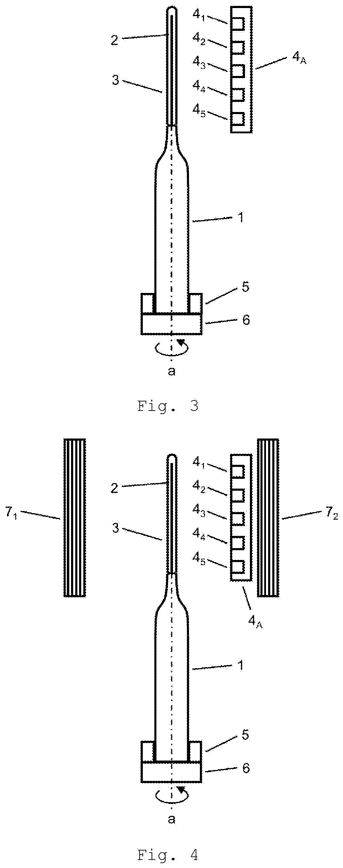

In Fig. 3 ist schematisch eine seitliche Ansicht einer Ausführungsvariante einer erfindungsgemässen Vorrichtung zur Inspektion von Spritzennadeln dargestellt. Die Spritze 1 ist dabei in einer Spritzenhalterung 5 eingespannt, die auf einer Dreh-/Rotationsvorrichtung 6 angeordnet ist, mittels deren die Spritze 1 um ihre Längsachse a rotiert werden kann. Durch die Rotation verändert die Kanüle 2 periodisch das Erdmagnetfeld in der Umgebung der Kanüle 2. Das Erdmagnetfeld wird mit Hilfe der Magnetfeldsensoranordnung 4_{A}, welche aus fünf Magnetfeldsensoren 4₁, ..., 4₅, besteht, die vertikal übereinander parallel zur Spritzenlängsachse a angeordnet sind, ausgemessen. Alternativ könnte auch ein einzelner Magnetfeldsensor eingesetzt werden, der entlang der Messstrecke S hoch oder runter bewegt wird.

Der zeitliche Verlauf des gemessenen magnetischen Feldes wird anschliessend in den Frequenzbereich transformiert. Dies kann für einzelne Frequenzen z.B. mittels diskreter Fouriertransformation erfolgen. Besonders relevant sind dabei die Rotationsfrequenz der Spritze 1 sowie deren zweite Harmonische (= doppelte Rotationsfrequenz). Wenn die Kanüle 2 einen Knick aufweist, so wird das magnetische Feld durch diesen einmal pro Umdrehung in Richtung der Magnetfeldsensoranordnung 4_{A}, d.h. näher an diese heran, gelenkt, sodass die magnetische Feldstärke bei der Rotationsfrequenz periodisch zu- und abnimmt. Die Verteilung des magnetischen Feldes entlang der Kanüle 2 wird daher bei der abgeknickten Spitze bei der Rotationsfrequenz ein grösseres Maximum aufweisen als bei einer geraden Kanüle 2. Auch wird sich aufgrund des Knicks eine andere Phasenlage des magnetischen Felds ergeben im Vergleich zu der Situation mit einer geraden Kanüle 2. Basierend auf der Amplitude und/oder Phasenlage des gemessenen magnetischen Feldes kann daher auf den Zustand der Kanüle 2 zurückgeschlossen werden, insbesondere wenn diese mit dem zuvor gemessenen magnetischen Feld in der Umgebung einer intakten, d.h. geraden Referenzkanüle 2 verglichen werden. Als Phasenlage kann dabei auch die Differenz der Phase des magnetischen Feldes an zwei beabstandeten Magnetfeldsensoren 4_{1...5} (z.B. zwei benachbarten Magnetfeldsensoren 4ᵢ & 4ᵢ₋₁ der Magnetfeldsensoranordnung 4_{A}) verwendet werden.

Bei den Messungen sollte darauf geachtet werden, dass magnetische Störfelder vermieden werden. So erzeugen z.B. elektrische Servomotoren solche Störfelder, welche mit der Drehzahl des Servomotors moduliert sind. Um deren Einfluss auf die Messungen zu minimieren, kann z.B. ein Getriebe eingesetzt werden, sodass die Spritze 1 um ein Vielfaches schneller oder langsamer dreht als der Servomotor. Ist die Drehzahl des Servomotors z.B. dreimal so hoch wie die Rotationsgeschwindigkeit der Spritze (d.h. Übersetzungsverhältnis 3:1), so wird das zu messende magnetische Feld bei der Rotationsfrequenz der Spritze 1 kaum gestört durch das Störfeld, welche durch den Servomotor bei der dreifachen Rotationsfrequenz der Spritze 1 erzeugt wird. Alternativ kann auch eine magnetische Abschirmung zur Unterdrückung von magnetischen Störfeldern eingesetzt wird.

Anstatt das Erdmagnetfeld für die Messungen zu verwenden, kann auch ein durch eine Anordnung mit einem oder mehreren Permanentmagneten gebildetes Magnetfeld, oder durch eine Anordnung mit einem oder mehreren Elektromagneten, wie z.B. einer Helmholtz-Spule, gebildetes Magnetfeld, benutzt werden, wobei die Anordnung insbesondere auch einen oder mehrere Eisenkerne aufweisen kann. Dadurch kann z.B. ein starkes homogenes Magnetfeld generiert werden.

In Fig. 4. ist schematisch eine seitliche Ansicht einer solchen Ausführungsvariante mit einer Helmholtz-Spule dargestellt. Dabei wird je eine Spule des Helmholtz-Spulenpaares 7₁, 7_ links und rechts der zu inspizierenden Kanüle 2 angeordnet. Zwischen den beiden Spulen 7₁, 7₂ bildet sich ein homogenes horizontal ausgerichtetes Magnetfeld, welches (wie in Fig. 3) mit der Magnetfeldsensoranordnung 4_{A} ausgemessen werden kann.

### LISTE DER BEZUGSZEICHEN

- 1: Spritze
- 2: Kanüle, Nadel
- 3: Kanülenschutzkappe
- 4_{1...5}: Magnetfeldsensor
- 4_{A}: Magnetfeldsensoranordnung mit mehreren Magnetfeldsensoren
- 5: Spritzenhalterung
- 6: Dreh-/Rotationsvorrichtung (ev. mit Hebevorrichtung)
- 7_{1,2}: Helmholtz-Spule
- a: Spritzenlängsachse
- F: magnetische Feldlinie
- S: Sensorlinie, Messstrecke

## Patentansprüche

1. Verfahren zur Inspektion eines Zustands einer auf einer Spritze (1) aufgesetzten Kanüle (2), welche sich in einer Kanülenschutzkappe (3) befindet, wobei das Verfahren folgende Schritte umfasst:
- Messen eines magnetischen Feldes, insbesondere einer magnetischen Feldverteilung, in einer Umgebung der Kanüle (2), und
- Bestimmen einer Verformung des magnetischen Feldes, insbesondere eines Feldlinienverlaufs, im Vergleich zum Fall einer geraden Kanüle.

2. Verfahren nach Anspruch 1, wobei zum Messen des magnetischen Feldes, insbesondere der magnetischen Feldverteilung, ein oder mehrere Magnetfeldsensoren (4_{1...5}, 4_{A}), insbesondere ein Induktionssensor, ein Fluxgate-Magnetometer, ein Hall-Sensor, ein magnetoresistiver Sensor, wie z.B. ein AMR-, CMR-, GMR- oder TMR-Sensor, eingesetzt werden.

3. Verfahren nach Anspruch 2, wobei der eine oder die mehreren Magnetfeldsensoren (4_{1...5}, 4_{A}) als ein-, zwei- oder drei-achsige Magnetfeldsensoren (4_{1...5}, 4_{A}) ausgeführt sind, um das magnetische Feld, insbesondere die magnetische Feldverteilung, ein-, zwei- oder drei-dimensional auszumessen.

4. Verfahren nach Anspruch 2 oder 3, wobei zum Messen des magnetischen Feldes, insbesondere der magnetischen Feldverteilung, die Kanüle (2) relativ zu dem einen oder den mehreren Magnetfeldsensoren (4_{1...5}, 4_{A}) bewegt wird, wobei die Spritze (1) insbesondere um ihre Längsachse (a) rotiert und/oder entlang ihrer Längsachse (a) bewegt wird, oder der eine oder die mehreren Magnetfeldsensoren (4_{1...5}, 4_{A}) parallel zur Längsachse (a) der Spritze (1) bewegt werden.

5. Verfahren nach einem der Ansprüche 1 bis 4, wobei das magnetische Feld, mindestens zum Teil durch das Erdmagnetfeld, durch eine Anordnung mit einem oder mehreren Permanentmagneten gebildetes Magnetfeld, oder durch eine Anordnung mit einem oder mehreren Elektromagneten, wie z.B. einer Helmholtz-Spule (7₁, 7₂), gebildetes Magnetfeld, erzeugt wird, wobei die Anordnung insbesondere auch einen oder mehrere Eisenkerne aufweist.

6. Verfahren nach einem der Ansprüche 1 bis 5, umfassend ein Vergleichen des gemessenen magnetischen Feldes, insbesondere der gemessenen magnetischen Feldverteilung, für die Kanüle (2) mit dem gemessenen magnetischen Feld, insbesondere der gemessenen magnetischen Feldverteilung, für eine Referenzspritze mit einer aufgesetzten Referenzkanüle, welche sich in einer Kanülenschutzkappe befindet, welche einen Sollzustand aufweist, und ein Bestimmen des Zustands der auf der Spritze (1) aufgesetzten Kanüle (2) basierend auf dem Vergleichen.

7. Verfahren nach Anspruch 6, wobei das Bestimmen des Zustands der auf der Spritze (1) aufgesetzten Kanüle (2) auf einem Vergleichen einer Amplitude und/oder Phasenlage des gemessenen magnetischen Feldes für die Referenzkanüle mit einer Amplitude und/oder Phasenlage des gemessenen magnetischen Feldes für die Kanüle (2) basiert.

8. Verfahren nach einem der Ansprüche 1 bis 7, umfassend ein Transformieren eines gemessenen zeitlichen Verlaufs des magnetischen Feldes, insbesondere der magnetischen Feldverteilung, vom Zeitbereich in den Frequenzbereich.

9. Vorrichtung zur Inspektion eines Zustands einer auf einer Spritze (1) aufgesetzten Kanüle (2), welche sich in einer Kanülenschutzkappe (3) befindet, wobei die Vorrichtung folgendes umfasst:
- ein oder mehrere Magnetfeldsensoren (4_{1...5}, 4_{A}), insbesondere ein Induktionssensor, ein Fluxgate-Magnetometer, ein Hall-Sensor, ein magnetoresistiver Sensor, wie z.B. ein AMR-, CMR-, GMR- oder TMR-Sensor, zum Messen eines magnetischen Feldes, insbesondere einer magnetischen Feldverteilung, in einer Umgebung der Kanüle (2); und
- eine Vergleichereinheit, wobei die Vergleichereinheit zum Vergleichen des gemessenen magnetischen Feldes, insbesondere der gemessenen magnetischen Feldverteilung, für die Kanüle (2) mit dem gemessenen magnetischen Feld, insbesondere der gemessenen magnetischen Feldverteilung, für eine Referenzspritze mit einer aufgesetzten Referenzkanüle, welche sich in einer Kanülenschutzkappe befindet, welche einen Sollzustand aufweist, ausgebildet ist, und wobei die Vergleichereinheit ausgebildet ist, den Zustand der auf der Spritze (1) aufgesetzten Kanüle (2) basierend auf dem Vergleich zu bestimmen.

10. Vorrichtung nach Anspruch 9, wobei der eine oder die mehreren Magnetfeldsensoren (4_{1...5}, 4_{A}) als ein-, zwei- oder drei-achsige Magnetfeldsensoren (4_{1...5}, 4_{A}) ausgeführt sind, um das magnetische Feld, insbesondere die magnetische Feldverteilung, ein-, zwei- oder drei-dimensional auszumessen.

11. Vorrichtung nach Anspruch 9 oder 10, weiter umfassend eine Einrichtung (6) zum Bewegen der Kanüle (2) relativ zu dem einen oder den mehreren Magnetfeldsensoren (4_{1...5}, 4_{A}), insbesondere zum Rotieren die Spritze (1) um ihre Längsachse (a) und/oder zum Fahren der Spritze (1) entlang ihrer Längsachse (a), oder zum Fahren des einen oder der mehreren Magnetfeldsensoren (4_{1...5}, 4_{A}) parallel zur Längsachse (a) der Spritze (1).

12. Vorrichtung nach einen der Ansprüche 9 bis 11, weiter umfassend eine Anordnung mit einem oder mehreren Permanentmagneten und/oder eine Anordnung mit einem oder mehreren Elektromagneten, wie z.B. einer Helmholtz-Spule (7₁, 7₂), zum Erzeugen eines Magnetfeldes, wobei die Anordnung insbesondere auch einen oder mehrere Eisenkerne aufweist.

13. Vorrichtung nach Anspruch 9, wobei die Vergleichereinheit ausgebildet ist, um einen Vergleich einer Amplitude und/oder Phasenlage des gemessenen magnetischen Feldes für die Referenzkanüle mit einer Amplitude und/oder Phasenlage des gemessenen magnetischen Feldes für die Kanüle auszuführen, und daraus den Zustand der auf der Spritze (1) aufgesetzten Kanüle (2) zu bestimmen.

14. Vorrichtung nach einem der Ansprüche 9 bis 13, weiter umfassend eine Einheit zur Ausführung einer Transformation eines gemessenen zeitlichen Verlaufs des magnetischen Feldes, insbesondere der magnetischen Feldverteilung, vom Zeitbereich in den Frequenzbereich.

15. Vorrichtung nach einen der Ansprüche 9 bis 14, weiter umfassend einen Ausgang, insbesondere zum Bereitstellen eines Ausgangssignals, welcher dazu ausgebildet ist, um den Zustand der Kanüle (2) als mindestens eines von gerade, verbogen, geknickt, gestaucht, ab-/gebrochen, koaxial zur Spritzenlängsachse (a), schräg zur Spritzenlängsachse (a), exzentrisch zur Spritzenlängsachse (a) anzugeben.

## Claims

1. Method for the inspection of a condition of a cannula (2) which is mounted on a syringe (1) and located in a cannula protective cap (3), wherein the method comprises the following steps:
- measuring of a magnetic field, in particular a magnetic field distribution, in the vicinity of the cannula (2), and
- determining a deformation of the magnetic field, in particular of the magnetic field distribution, compared to the case of a straight cannula.

2. Method according to claim 1, wherein one or more magnetic field sensors (4_{1...5}, 4_{A}), in particular an induction sensor, a fluxgate magnetometer, a Hall sensor, a magnetoresistive sensor, such as an AMR, CMR, GMR or TMR sensor, are used to measure the magnetic field, in particular the magnetic field distribution.

3. Method according to claim 2, wherein the one or more magnetic field sensors (4_{1...5}, 4_{A}) are implemented as one-, two- or three-axis magnetic field sensors (4_{1...5}, 4_{A}) to measure the magnetic field, in particular the magnetic field distribution, in one, two or three dimensions.

4. Method according to claim 2 or 3, wherein for measuring the magnetic field, in particular the magnetic field distribution, the cannula (2) is moved relative to the one or more magnetic field sensors (4_{1...5}, 4_{A}), wherein the syringe (1) is in particular rotated about its longitudinal axis (a) and/or moved along its longitudinal axis (a), or the one or more magnetic field sensors (4_{1...5}, 4_{A}) are moved parallel to the longitudinal axis (a) of the syringe (1).

5. Method according to one of claims 1 to 4, wherein the magnetic field is generated, at least in part by the earth's magnetic field, by an arrangement comprising one or more permanent magnets, or by an arrangement comprising one or more electromagnets, such as a Helmholtz coil (7₁, 7₂), wherein the arrangement in particular also comprises one or more iron cores.

6. Method according to one of claims 1 to 5, comprising a comparison of the measured magnetic field, in particular the measured magnetic field distribution, for the cannula (2) with the measured magnetic field, in particular the measured magnetic field distribution, for a reference syringe with a mounted reference cannula located in a cannula protective cap having a desired condition, and a determination of the condition of the cannula (2) mounted on the syringe (1) based on the comparison.

7. Method according to claim 6, wherein the determination of the condition of the cannula (2) mounted on the syringe (1) is based on the comparison of an amplitude and/or phase position of the measured magnetic field for the reference cannula with an amplitude and/or phase position of the measured magnetic field for the cannula (2).

8. Method according to one of claims 1 to 7, comprising a transformation of a measured time course of the magnetic field, in particular the magnetic field distribution, from the time domain to the frequency domain.

9. Device for the inspection of a condition of a cannula (2) mounted on a syringe (1), which is located in a cannula protective cap (3), wherein the device comprises the following:
- one or more magnetic field sensors (4_{1...5}, 4_{A}), in particular an induction sensor, a fluxgate magnetometer, a Hall sensor, a magnetoresistive sensor, such as an AMR, CMR, GMR or TMR sensor, for measuring a magnetic field, in particular a magnetic field distribution, in the vicinity of the cannula (2); and
- a comparator unit, wherein the comparator unit is adapted to compare the measured magnetic field, in particular the measured magnetic field distribution, for the cannula (2) with the measured magnetic field, in particular the measured magnetic field distribution, for a reference syringe with a mounted reference cannula located in a cannula protective cap having a desired condition, and wherein the comparator unit is adapted to determine the condition of the cannula (2) mounted on the syringe (1) based on the comparison.

10. Device according to claim 9, wherein the one or more magnetic field sensors (4_{1...5}, 4_{A}) are implemented as one-, two- or three-axis magnetic field sensors (4_{1...5}, 4_{A}) to measure the magnetic field, in particular the magnetic field distribution, in one, two or three dimensions.

11. Device according to claim 9 or 10, further comprising a means (6) for moving the cannula (2) relative to the one or more magnetic field sensors (4_{1...5}, 4_{A}), in particular for rotating the syringe (1) about its longitudinal axis (a) and/or for moving the syringe (1) along its longitudinal axis (a), or for moving the one or more magnetic field sensors (4_{1...5}, 4_{A}) parallel to the longitudinal axis (a) of the syringe (1).

12. Device according to one of claims 9 to 11, further comprising an arrangement having one or more permanent magnets and/or an arrangement having one or more electromagnets, such as a Helmholtz coil (7₁, 7₂), for generating a magnetic field, wherein the arrangement in particular also comprises one or more iron cores.

13. Device according to claim 9, wherein the comparator unit is adapted to perform a comparison of an amplitude and/or phase position of the measured magnetic field for the reference cannula with an amplitude and/or phase position of the measured magnetic field for the cannula (2), and to determine therefrom the condition of the cannula (2) mounted on the syringe (1).

14. Device according to one of claims 9 to 13, further comprising a unit for performing a transformation of a measured time course of the magnetic field, in particular the magnetic field distribution, from the time domain to the frequency domain.

15. Device according to one of claims 9 to 14, further comprising an output, in particular for providing an output signal, which is adapted to indicate the condition of the cannula (2) as at least one of straight, bent, kinked, compressed, broken/severed, coaxial to the syringe longitudinal axis (a), oblique to the syringe longitudinal axis (a), eccentric to the syringe longitudinal axis (a).

## Revendications

1. Procédé pour vérifier l'état d'une canule (2) installée sur une seringue (1) et se trouvant dans un capuchon de protection de canule (3), le procédé comprenant les étapes suivantes consistant à :
- mesurer un champ magnétique, en particulier une répartition de champ magnétique, dans l'environnement de la canule (2), et
- déterminer une déformation du champ magnétique, en particulier du tracé des lignes de champ, par rapport à une canule droite.

2. Procédé selon la revendication 1, dans lequel pour mesurer le champ magnétique, en particulier la répartition de champ magnétique, on utilise un ou plusieurs capteurs de champ magnétique (4_{1...5}, 4_{A}), en particulier un capteur inductif, un magnétomètre Fluxgate, un capteur à effet Hall, un capteur magnéto-résistif, tel qu'un capteur AMR, CMR, GMR ou TMR.

3. Procédé selon la revendication 2, dans lequel ledit ou lesdits plusieurs capteurs de champ magnétique (4_{1...5}, 4_{A}) sont réalisés sous la forme de capteurs de champ magnétique à un, deux ou trois axes (4_{1...5}, 4_{A}) pour mesurer le champ magnétique, en particulier la répartition de champ magnétique, en une, deux ou trois dimensions.

4. Procédé selon la revendication 2 ou 3, dans lequel pour mesurer le champ magnétique, en particulier la répartition de champ magnétique, on déplace la canule (2) par rapport audit ou auxdits plusieurs capteurs de champ magnétique (4_{1...5}, 4_{A}), la seringue (1) étant en particulier tournée autour de son axe longitudinal (a) et/ou déplacée le long de son axe longitudinal (a), ou ledit ou lesdits plusieurs capteurs de champ magnétique (4_{1...5}, 4_{A}) étant déplacés parallèlement à l'axe longitudinal (a) de la seringue (1).

5. Procédé selon l'une des revendications 1 à 4, dans lequel le champ magnétique est généré au moins en partie par le champ magnétique terrestre, par le champ magnétique formé par l'agencement d'un ou de plusieurs aimants permanents, ou par un champ magnétique formé par l'agencement d'un ou de plusieurs électro-aimants, comme par exemple une bobine de Helmholtz (7₁, 7₂), l'agencement présentant également en particulier un ou plusieurs noyaux de fer.

6. Procédé selon l'une des revendications 1 à 5, consistant à comparer le champ magnétique mesuré, en particulier la répartition de champ magnétique mesurée, pour la canule (2), avec le champ magnétique mesuré, en particulier la répartition de champ magnétique mesurée, pour une seringue de référence avec une canule de référence installée dessus et se trouvant dans un capuchon de protection de canule, qui présente un état de référence, et à déterminer l'état de la canule (2) installée sur la seringue (1) sur la base de cette comparaison.

7. Procédé selon la revendication 6, dans lequel la détermination de l'état de la canule (2) installée sur la seringue (1) est basée sur la comparaison de l'amplitude et/ou de la position de phase du champ magnétique mesuré pour la canule de référence avec l'amplitude et/ou la position de phase du champ magnétique mesurée pour la canule (2).

8. Procédé selon l'une des revendications 1 à 7, consistant à transformer un tracé temporel mesuré du champ magnétique, en particulier de la répartition de champ magnétique, en le faisant passer du temps à la fréquence.

9. Dispositif pour vérifier l'état d'une canule (2) installée sur une seringue (1) et se trouvant dans un capuchon de protection de canule (3), le dispositif comprenant les éléments suivants :
- un ou plusieurs capteurs de champ magnétique (4_{1...5}, 4_{A}), en particulier un capteur inductif, un magnétomètre Fluxgate, un capteur à effet Hall, un capteur magnéto-résistif, comme par exemple un capteur AMR, CMR, GMR ou TMR, pour mesurer un champ magnétique, en particulier une répartition de champ magnétique, dans l'environnement de la canule (2) ; et
- une unité de comparaison, l'unité de comparaison étant conçue pour comparer le champ magnétique mesuré, en particulier la répartition de champ magnétique mesurée, pour la canule (2) avec le champ magnétique mesuré, en particulier la répartition de champ magnétique mesurée, pour une seringue de référence avec une canule de référence se trouvant dans un capuchon de protection de canule et qui présente un état de référence, et l'unité de comparaison étant conçue pour déterminer l'état de la canule (2) installée sur la seringue (1) sur la base de cette comparaison.

10. Dispositif selon la revendication 9, dans lequel ledit ou lesdits plusieurs capteurs de champ magnétique (4_{1...5}, 4_{A}) sont réalisés sous la forme de capteurs de champ magnétique (4_{1...5}, 4_{A}) à un, deux ou trois axes, pour mesurer le champ magnétique, en particulier la répartition de champ magnétique, en une, deux ou trois dimensions.

11. Dispositif selon la revendication 9 ou 10, comprenant en outre un équipement (6) pour déplacer la canule (2) par rapport audit ou auxdits plusieurs capteurs de champ magnétique (4_{1...5}, 4_{A}), en particulier pour faire tourner la seringue (1) autour de son axe longitudinal (a) et/ou pour déplacer la seringue (1) le long de son axe longitudinal (a), ou pour déplacer ledit ou lesdits plusieurs capteurs de champ magnétique (4_{1...5}, 4_{A}) parallèlement à l'axe longitudinal (a) de la seringue (1).

12. Dispositif selon l'une des revendications 9 à 11, comprenant en outre un agencement d'un ou de plusieurs aimants permanents et/ou un agencement d'un ou de plusieurs électro-aimants, comme par exemple une bobine de Helmholtz (7₁, 7₂), pour générer un champ magnétique, l'agencement présentant également en particulier un ou plusieurs noyaux de fer.

13. Dispositif selon la revendication 9, dans lequel l'unité de comparaison est conçue pour comparer l'amplitude et/ou la position de phase du champ magnétique mesuré pour la canule de référence avec l'amplitude et/ou la position de phase du champ magnétique mesuré pour la canule, et à définir sur cette base l'état de la canule (2) installée sur la seringue (1).

14. Dispositif selon l'une des revendications 9 à 13, comprenant en outre une unité pour transformer un tracé temporel mesuré du champ magnétique, en particulier de la répartition de champ magnétique, du temps en fréquence.

15. Dispositif selon l'une des revendications 9 à 14, comprenant en outre une sortie, en particulier pour fournir un signal de sortie, qui est conçue pour indiquer si la canule (2) est droite, déformée, coudée, comprimée, cassée, coaxiale par rapport à l'axe longitudinal de seringue (a), oblique par rapport à l'axe longitudinal de seringue (a), excentrique par rapport à l'axe longitudinal de seringue (a) .
